# EUROPEAN PATENT SPECIFICATION

(11) **EP 2 663 530 B1**
(45) Date of publication and mention of the grant of the patent: **17.12.2014**
(21) Application number: 12704122.6
(22) Date of filing: 16.01.2012
(51) Int. Cl.: C02F 1/461, C02F 1/467, C02F 1/72, C02F 103/42, C02F 103/02

(54) **WATER PURIFICATION**
WASSERREINIGUNG
PURIFICATION D'EAU

(30) Priority: 14.01.2011 ZA 201100380
(43) Date of publication of application: 20.11.2013
(73) Proprietor: Wet Trust, 7655 Wellington (ZA)
(72) Inventor: FAURE, Frederik, Jacobus, 7646 Paarl (ZA)
(74) Representative: Cabinet Plasseraud
(86) International application number: PCT/IB2012/050191
(87) International publication number: WO 2012/095828

(56) References cited:
- WO-A1-98/31636
- JP-A- 63 051 991
- US-A- 4 048 032
- US-A- 4 098 660
- US-A- 4 451 341
- US-A1- 2008 314 762

## Description

### BACKGROUND OF THE INVENTION

This invention relates to a method of purifying water and apparatus for use in such method.

Of all the water on the Earth, about 97% exists in the ocean as saltwater. The remaining 3% represents the amount of freshwater on the planet. Unfortunately, 90% of this freshwater is trapped in glaciers and ice caps, and in general humans cannot extract it for use. Ultimately, only 0.014% of the earth's total volume of water is easily available for agricultural, industrial, and domestic purposes. This water exists in a variety of forms, including soil moisture, groundwater, water vapour, lakes, dams and streams. This water is generally in a state which is not suitable for the above purposes.

It is an object of this invention to provide a method for enhancing the quality of water for agricultural, industrial, and domestic purposes. Some known water treatment methods similar to the method of the present invention are disclosed in the prior art documents US2008/314762 and WO97/19896.

### SUMMARY OF THE INVENTION

In a first aspect of the invention there is provided a method for purifying water, wherein:
oxygen is introduced into the water by electrolysis of the water; and
the water is treated with silver ions produced at a silver electrode/s supplied with electric current, and copper ions produced at copper electrode/s supplied with electric current.

The electrolysis of the water takes place by passing an electric current through paired electrodes made from carbon, such as graphite, or an inert metal, such as platinum, stainless steel or titanium, preferably stainless steel.

The water is treated with ionized silver and copper, preferably ionized silver, copper and zinc produced at silver, copper and zinc electrodes that are supplied with electric current.

The electric current supplied to the electrolysis electrodes and transition metal electrodes may be from 300mA to 3A, with the current to each eletrode pair separately controlled.

Preferably, the water is maintained at a pressure of from 0.6 bar to 10 bar (6x10⁴ Pa to 1x10⁶ Pa), preferably from 2 to 6 bar (2x10⁵ Pa to 6x10⁵ Pa).

In a second aspect of the invention, there is provided an apparatus for treating water, the apparatus comprising:
means for introducing oxygen into the water by electrolysis of the water;
silver electrode/s for introducing silver ions into the water by electrolysis; and copper electrode/s for introducing copper ions into the water by electrolysis.

The means for introducing oxygen into the water by electrolysis of the water comprise paired electrodes made from carbon, such as graphite, or an inert metal, such as platinum, stainless steel or titanium, preferably stainless steel, through which an electric current is passed.

The means for introducing ionized silver and copper into the water comprise an electrode made from the transition metal, through which a current is passed. The apparatus according to the present invention comprises silver and copper electrodes, preferably silver, copper and zinc electrodes.

The transition metal electrodes may be connected, thereby forming electrode pairs. Each electrode pair may be connected to a DC power supply which is able to supply an electric current from 0-7Amp separately to each electrode pair, with no limit on voltage. Preferably, the power supply is connected to a common power source, which may be a 220 to 240V AC or solar panel with polarity switching in a time frame of 3 - 10 minutes. The electrodes in the electrode pair may be placed apart at a distance of 18mm or less, preferably at a distance of 8-10mm.

Preferably, the means for introducing oxygen into the water by electrolysis of the water; and means for introducing ionized silver and copper into the water are housed in a vessel having a water inlet and a water outlet, for example a hollow pipe that may have a length of 0.5 - 3m and a diameter of 50 - 350 mm, preferably length of 1 - 1.5m and a diameter of 100-150mm.

### BRIEF DESCRIPTION OF THE DRAWINGS

- **Figure 1**: is a diagram of the apparatus of the invention.
- **Figure 2**: is a cross sectional diagram of a preferred embodiment of the apparatus of the invention.
- **Figure 3**: is a schematic representation of an arrangement of the preferred embodiment of the apparatus of the present invention.

### DESCRIPTION OF A PREFERRED EMBODIMENT OF THE INVENTION

The invention allows for water to be disinfected by using the oligodynamic effect of transition metal ions on microorganisms in combination with the oxidative effect of nascent oxygen on bacterial enzymes and other organic material.

Transition Nonferrous Metal Ionization (TNMI) is an algaecide and biocide with successes in the bacteria, virus and fungi treatment. Not only does TNMI enhance water quality with a long term disinfection action, it also enhances the mineral content with specific benefits to human, plants and animals.

The oligodynamic effect of metal ions was discovered in 1893 by the Swiss Karl Wilhelm von Nägeli as a toxic effect of metal ions on living cells, algae, molds, spores, fungus, virus, prokaryotic and eukaryotic microorganisms, even in relatively low concentrations. This antimicrobial effect is shown by ions of: mercury, silver, copper, iron, lead, zinc, bismuth, gold, aluminum and other metals.

Metal ions, especially those of heavy metals, show this effect. The exact mechanism of action is still unknown. Data from silver suggest that these ions denature enzymes of the target cell or organism by binding to reactive groups, resulting in their precipitation and inactivation. Silver inactivates enzymes by reacting with the thiol groups to form silver sulfides. Silver also reacts with the amino-, carboxyl-, phosphate-, and imidazole-groups and diminish the activities of lactate dehydrogenate and glutathione peroxides.

Nascent oxygen oxidizes bacterial enzymes and other organic material. The reaction of nascent oxygen with bacterial enzymes and other organic material is instantaneous.

Metallic silver is one of the metals which are able to produce nascent oxygen. Among all the metals, silver is unique in its behaviour with oxygen. It is known that molecular oxygen is adsorbed on the surface of silver in its atomic state. Also, atomic oxygen diffuses more freely within silver than any other metal. Davies L. R et. al. (*The development of function of silver in water purification and disease control*), state that atomic oxygen fits very well in the octahedral holes of gold, silver, and copper. In gold, the electron cloud of oxygen tends to be repelled by the lattice electrons of the gold atoms stopping movement through the holes. With copper, the oxide is formed resulting in a barrier. Silver, with an almost perfect fit, offers so little repulsion that minimum thermal energy is required to move oxygen through the silver lattice, thereby producing nascent oxygen.

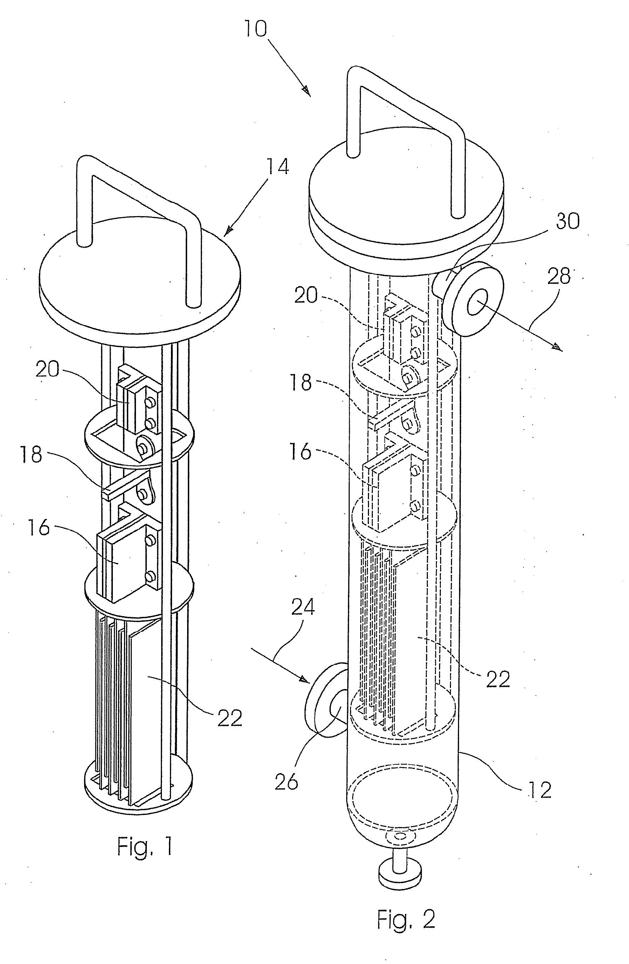

Referring to Figures 1 and 2, water is treated in a Transition Nonferrous Metal Ionization (TNMI) unit (10) comprising a vessel (12) in the form of a hollow pipe having a length of 1.2m and a diameter of 110mm, and an assembly (14) comprising a silver electrode (16), copper electrode (18) and zinc electrode (20) located inside the vessel (12). Each transition metal electrode is connected to another electrode, thereby forming separate electrode pairs. Electrodes in an electrode pair are preferably placed at a distance of 13mm or less from each other preferably at a distance of 9mm. Additional multi-stack electrodes may be added if needed, depending on the needs to be achieved. The assembly also contains a stainless steel electrode(s) (22), for electrolysis of water. Each electrode pair may be connected to a DC power supply, which is connected to a common 220V AC power source or solar panel, with polarity switching in a time frame of 3 - 10 minutes. The electrical current to each eletrode pair is controlled separately.

Water to be treated (24) is supplied to the unit from a source via an inlet (26) to the vessel (12). The transition metal electrodes are ionized by supplying current to the electrode pair supplied directly to the electrode pairs from an external power supply. Typically molecular oxygen is present in the water that is being treated, although its concentration may vary depending on the factors such as the source of the water and the amount of minerals in water. Distilled water can absorb more oxygen than well waters with higher mineral content. For the same reason, sea water holds less dissolved water than fresh water. Oxygen is introduced into the water by electrolysis of water, using the stainless steel electrode(s) (22). Graphite, platinum, or titanium electrodes may also be used for this purpose. During electrolysis, water (H₂O) is decomposed into oxygen (O₂) and hydrogen gas (H₂) as a result of an electric current being passed through the water. A DC electrical power source is connected to multiple electrodes, typically made from graphite or an inert metal such as platinum, stainless steel or titanium, which are placed in the water. In an optimally designed unit, hydrogen will appear at the cathode (the negatively charged electrode, from where electrons are released into the water), and oxygen will appear at the anode (the positively charged electrode). Assuming ideal faraday efficiency the generated amount (moles) of hydrogen is twice that of oxygen, and both are proportional to the total electrical charge that was sent through the solution. Without wishing to be bound by theory, it is believed that oxygen produced by electrolysis is generated in its highly reactive, nascent form of a single atom. Because of its instability in nascent form, individual oxygen atoms quickly combine in pairs to form stable molecules of O₂. Electrolytic produce nascent oxygen, during its short lifetime, is a more potent oxidizer of bacteria than dissolved O₂, and hence a lower concentration is tolerable.

After electrolysis, the water comes into contact with the silver electrode (16), where ionized silver disinfects the water using the oligodynamic effect. The silver electrode also serves the purpose of producing nascent oxygen from the molecular oxygen already available in the water, or molecular oxygen made available in the water by injection of oxygen or the electrolysis of the water, or a combination of both methods. The available molecular oxygen is readily adsorbed on the surface of, and diffuses through the lattice of, the silver as nascent oxygen. The nascent oxygen instantaneously oxidizes the bacterial enzymes and other organic material in the water. If necessary, oxygen may be injected into the water (24), via an injector connected in series to the TNMI unit (1), prior to the water entering the TNMI unit (10). The water is further brought into contact with the copper and zinc electrodes, where it is further treated using the oligodynamic effect of ionized copper and zinc. The treated water (28) leaves the TNMI unit (10) via the outlet (30). Typically the water would be maintained at a pressure sufficient to maintain the supplied oxygen dissolved therein. The TNMI Unit is operated at a pressure of between 0.6 bar to 10 bar (6x10⁴ Pa to 1x10⁶ Pa and at a flow rate of 15000 to 25000, typically about 17000 to 20000 litres of water per hour.

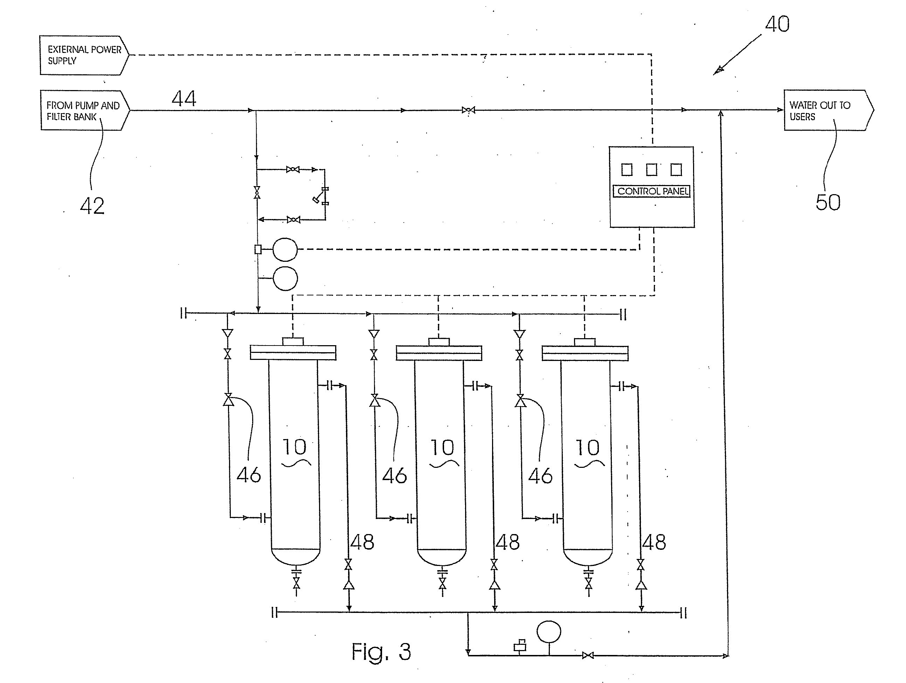

Referring to figure 3, the apparatus (40) of the present invention may consist of more than one TNMI Units (10) connected in parallel. The TNMI units may also be connected in series. Water to be treated (44) is fed from a source (42) to each TMNI unit (10) where it is treated as described above. Prior to entering the TNMI unit, oxygen is injected into the water via the injector (46), connected in-line with the TNMI unit (10). The treated water (48) is collected and supplied to a user access point (50), such as a tap, a swimming pool etc.

The apparatus also comprises a 220V AC (13) main power supply, and each TNMI unit contains a DC power supply (13) connected to each electrode pair, preferably with polarity switching, and which is able to change the current from 0-7amp, within 3 to 10 minutes, per electrode pair, with no limit on voltage. The apparatus may also comprise indicators and alarms incorporated into the unit to indicate mains to the unit, DC to the TNMI unit, as well as current (Amp) status to each electrode pair.

The invention can be used in various applications such as pools, spas, fountains, Heating, Ventilating, and Air Conditioning (HVAC) equipment, cooling towers, wine industry, influent & effluent treatment, hospitals, food and potable water systems, irrigation systems, and various domestic, agricultural and industrial applications.

The invention can be used as a replacement of chlorine based water disinfection methods and still ensure primary and secondary disinfection abilities. It is also envisaged that the invention may be used in conjunction with other environmentally friendly water disinfection methods in order to ensure very high quality pathologically potable water results.

By preventing and combating growth of bacteria fungal and viral pathogens in water, the invention provides a non-toxic and environmentally friendly method of ensuring the public health in both public and private applications.

### EXAMPLE

Dam water from a site in Grabouw in South Africa was treated in a TNMI unit of the invention using a combination of ionization with copper, silver, zinc with electrolysis of oxygen.

Table 1 shows the results of an analysis of a sample of the water treated by the apparatus 10 described above with the vessel 12 having a length of 1.2m and an internal diameter of 110mm, by an independent laboratory.

The apparatus 10 was operated under the following conditions:
Pressure: 4 bar
Water flow rate: 19500 litres per hour
Power supply to electrodes : Single 12VDC with a current supply between 300mA to 3A with polarity switching to each electrode pair.

The analysis showed a significant reduction in bacterial, fungal and viral pathogens within 2 seconds of starting the treatment.

**Table 1**

| Source | Lab. | Total Bacteria | Coliforms | E Coli | | Source | Lab. | Ag |
|---|---|---|---|---|---|---|---|---|
| | No. | /1 ml | /100ml | /100ml | | | No. | mg/l |
| Dam | 5407 | 19200 | >2420 | 44 | | Dam | 5407 | 0 |
| 2s after | 5408 | 200 | N/D | N/D | | 2s After | 5408 | 0.025 |
| Treat Resv | 5409 | 200 | 4 | N/D | | | | |
| Max. allowed | | 5000 | 10 | 0 | | | | |

## Claims

1. A method for purifying water, wherein:
oxygen is introduced into the water by electrolysis of the water wherein the electrolysis of the water takes place by passing an electric current through paired electrodes made from carbon, or an inert metal; and
the water is treated with:
silver ions produced at a silver electrode/s supplied with electric current, and
copper ions produced at copper electrode/s supplied with electric current.

2. The method as claimed in claim 1, wherein the inert metal is platinum, stainless steel or titanium, preferably stainless steel.

3. The method as claimed in claim 2, wherein the water is treated with ionized silver, ionized copper and ionized zinc produced at silver, copper and zinc electrodes that are supplied with electric current.

4. The method as claimed in any one of claims 1 to 3, wherein oxygen is injected into the water prior to the treatment of the water by electrolysis.

5. An apparatus for treating water, the apparatus comprising:
means for introducing oxygen into the water by electrolysis of the water wherein the means for introducing oxygen into the water by electrolysis of the water comprises paired electrodes made from carbon, or an inert metal, through which an electric current may be passed;
silver electrode/s for introducing silver ions into the water by electrolysis; and
copper electrode/s for introducing copper ions into the water by electrolysis.

6. The apparatus as claimed in claim 5, wherein the inert metal is platinum, stainless steel or titanium, preferably stainless steel.

7. The apparatus as claimed in claim 5 or 6, further comprising zinc electrode/s for introducing zinc ions into the water by electrolysis.

8. The apparatus as claimed in any one of claims 5 to 7, wherein the silver, copper and zinc electrodes are connected in electrode pairs.

9. The apparatus as claimed in claim 8, wherein the electrode pairs are placed apart at a distance of 18mm or less, preferably 8-10mm.

10. The apparatus as claimed in any one of claims 5 to 9, wherein the electrodes are housed in a vessel having a water inlet and a water outlet.

11. The apparatus as claimed in claim 10, wherein the vessel is a hollow pipe.

12. The apparatus as claimed in claim 11, wherein the hollow pipe has a length of 0.5 - 3m and a diameter of 50 - 350 mm.

13. The apparatus as claimed in claim 12, wherein the hollow pipe has a length of 1 - 1.5m and a diameter of 100-150mm.

14. The apparatus as claimed in any one of claims 5 to 13, comprising a DC power supply which supplies an electric current to each electrode pair separately.

15. The apparatus as claimed in any one of claims 5 to 14, comprising means for injecting oxygen into the water prior to the electrolysis of the water.

## Patentansprüche

1. Verfahren zum Reinigen von Wasser, wobei:
Sauerstoff mittels Elektrolyse des Wassers in das Wasser eingeleitet wird, wobei die Elektrolyse des Wassers mittels Durchleitung eines elektrischen Stroms durch gepaarte Elektroden aus Kohlenstoff oder einem inerten Metall erfolgt; und
das Wasser behandelt wird mit:
Silberionen, erzeugt an einer Silberelektrode/Silberelektroden, die mit elektrischem Strom versorgt ist/sind, und
Kupferionen, erzeugt an einer Kupferelektrode/Kupferelektroden, die mit elektrischem Strom versorgt ist/sind.

2. Verfahren nach Anspruch 1, wobei das inerte Metall Platin, rostfreier Stahl oder Titan ist, vorzugsweise rostfreier Stahl.

3. Verfahren nach Anspruch 2, wobei das Wasser mit ionisiertem Silber, ionisiertem Kupfer und ionisiertem Zink behandelt wird, das an mit elektrischem Strom versorgten Silber-, Kupfer- und Zinkelektroden erzeugt wird.

4. Verfahren nach einem der Ansprüche 1 bis 3, wobei Sauerstoff in das Wasser vor der Behandlung des Wassers mit Elektrolyse injiziert wird.

5. Vorrichtung zur Behandlung von Wasser, wobei die Vorrichtung umfasst:
Mittel zum Einleiten von Sauerstoff in das Wasser mittels Elektrolyse des Wassers, wobei die Mittel zum Einleiten von Sauerstoff in das Wasser mittels Elektrolyse des Wassers gepaarte Elektroden aus Kohlenstoff oder einem inerten Metall umfassen, durch welche ein elektrischer Strom geleitet werden kann;
eine Silberelektrode/Silberelektroden zum Einleiten von Silberionen in das Wasser mittels Elektrolyse; und
eine Kupferelektrode/Kupferelektroden zum Einleiten von Kupferionen in das Wasser mittels Elektrolyse.

6. Vorrichtung nach Anspruch 5, wobei das inerte Metall Platin, rostfreier Stahl oder Titan ist, vorzugsweise rostfreier Stahl.

7. Vorrichtung nach Anspruch 5 oder 6, weiter umfassend eine Zinkelektrode/Zinkelektroden zum Einleiten von Zinkionen in das Wasser mittels Elektrolyse.

8. Vorrichtung nach einem der Ansprüche 5 bis 7, wobei die Silber-, Kupfer und Zinkelektroden in Elektrodenpaaren miteinander verbunden sind.

9. Vorrichtung nach Anspruch 8, wobei die Elektrodenpaare in einem Abstand von 18 mm oder weniger, vorzugsweise 8 bis 10 mm, voneinander getrennt angeordnet sind.

10. Vorrichtung nach einem der Ansprüche 5 bis 9, wobei die Elektroden in einem Gefäß aufgenommen sind, das einen Wassereinlass und einen Wasserauslass aufweist.

11. Vorrichtung nach Anspruch 10, wobei das Gefäß ein hohles Rohr ist.

12. Vorrichtung nach Anspruch 11, wobei das hohle Rohr eine Länge von 0,5 bis 3 m und einen Durchmesser von 50 bis 350 mm aufweist.

13. Vorrichtung nach Anspruch 12, wobei das hohle Rohr eine Länge von 1 bis 1,5 m und einen Durchmesser von 100 bis 150 mm aufweist.

14. Vorrichtung nach einem der Ansprüche 5 bis 13, umfassend eine Gleichstromquelle, die jedem Elektrodenpaar getrennt einen elektrischen Strom bereitstellt.

15. Vorrichtung nach einem der Ansprüche 5 bis 14, umfassend Mittel zum Injizieren von Sauerstoff in das Wasser vor der Elektrolyse des Wassers.

## Revendications

1. Procédé pour purifier de l'eau, dans lequel :
de l'oxygène est introduit dans l'eau par électrolyse de l'eau, dans lequel l'électrolyse de l'eau a lieu en faisant passer un courant électrique à travers des électrodes appariées faites de carbone, ou d'un métal inerte ; et
l'eau est traitée avec :
- des ions argent produits au niveau d'une électrode/d'électrodes en argent alimentée(s) en courant électrique, et
- des ions cuivre produits au niveau d'une électrode/d'électrodes en cuivre alimentée(s) en courant électrique.

2. Procédé selon la revendication 1, dans lequel le métal inerte est du platine, de l'acier inoxydable ou du titane, de préférence de l'acier inoxydable.

3. Procédé selon la revendication 2, dans lequel l'eau est traitée avec de l'argent ionisé, du cuivre ionisé et du zinc ionisé produits au niveau d'électrodes en argent, cuivre et zinc qui sont alimentées en courant électrique.

4. Procédé selon une quelconque des revendications 1 à 3, dans lequel l'oxygène est injecté dans l'eau avant le traitement de l'eau par électrolyse.

5. Appareil pour traiter de l'eau, l'appareil comprenant :
des moyens pour introduire de l'oxygène dans l'eau par électrolyse de l'eau dans lequel les moyens pour introduire de l'oxygène dans l'eau par électrolyse de l'eau comprennent des électrodes appariées faites de carbone, ou d'un métal inerte, à travers lesquelles un courant électrique peut passer ;
une ou des électrode(s) en argent pour introduire des ions argent dans l'eau par électrolyse ; et
une ou des électrode(s) en cuivre pour introduire des ions cuivre dans l'eau par électrolyse.

6. Appareil selon la revendication 5, dans lequel le métal inerte est du platine, de l'acier inoxydable ou du titane, de préférence de l'acier inoxydable.

7. Appareil selon la revendication 5 ou 6, comprenant en outre une ou des électrode(s) en zinc pour introduire des ions zinc dans l'eau par électrolyse.

8. Appareil selon une quelconque des revendications 5 à 7, dans lequel les électrodes en argent, en cuivre et en zinc sont connectées en paires d'électrodes.

9. Appareil selon la revendication 8, dans lequel les paires d'électrodes sont placées de façon séparée à une distance de 18 mm ou moins, de préférence 8 à 10 mm.

10. Appareil selon une quelconque des revendications 5 à 9, dans lequel les électrodes sont logées dans une cuve comportant une entrée d'eau et une sortie d'eau.

11. Appareil selon la revendication 10, dans lequel la cuve est un tuyau creux.

12. Appareil selon la revendication 11, dans lequel le tuyau creux possède une longueur de 0,5 à 3 m et un diamètre de 50 à 350 mm.

13. Appareil selon la revendication 12, dans lequel le tuyau creux possède une longueur de 1 à 1,5 m et un diamètre de 100 à 150 mm.

14. Appareil selon une quelconque des revendications 5 à 13, comprenant une alimentation électrique CC qui fournit un courant électrique à chaque paire d'électrodes séparément.

15. Appareil selon une quelconque des revendications 5 à 14, comprenant des moyens pour injecter de l'oxygène dans l'eau avant l'électrolyse de l'eau.
